# EUROPEAN PATENT APPLICATION

(11) **EP 3 854 384 A1**
(43) Date of publication of application: **28.07.2021**
(21) Application number: 20461508.2
(22) Date of filing: 27.01.2020
(51) Int. Cl.: A61K 9/107, A61K 31/58, A61K 9/16, A61K 9/20, A61K 9/48, A61K 47/02, A61K 47/14, A61P 35/00

(54) **PHARMACEUTICAL FORMULATION COMPRISING ABIRATERONE ACETATE**

(71) Applicant: Adamed Pharma S.A., 05-152 Czosnow (PL)
(72) Inventor: WOS-LATOSI, Katarzyna, 05-092 Lomianki (PL); GARBERA, Kamil, 59-220 Legnica (PL)
(74) Representative: Sulikowski, Daniel

(57) **Abstract**

The present invention relates to a pharmaceutical formulation comprising a solidified self-microemulsifyng drug delivery system (SEDDS) of Abiraterone acetate, wherein the system is completely absorbed into a mesoporous material that acts as a functional carrier for the system creating a stable solid oral formulation comprising Abiraterone acetate in a non-crystalline form, and a process of preparing thereof. The pharmaceutical formulation can be used for manufacturing stable solid oral dosage forms of Abiraterone acetate.

## Description

### Field of the invention

The present invention relates to a solidified self-microemulsifyng drug delivery system (SEDDS) formulation comprising Abiraterone acetate in a non-crystalline form and to solid oral dosage forms comprising thereof. Moreover, provided is a process of preparing the solidified self-microemulsifyng drug delivery system (SEDDS) formulation comprising Abiraterone acetate in a non-crystalline form, and a solid oral dosage form comprising the pharmaceutical formulation comprising solidified self-microemulsifyng drug delivery system (SEDDS) of Abiraterone acetate as an active ingredient in a non-crystalline form absorbed into a mesoporous material and additional pharmaceutically acceptable excipients.

### Background of the invention

Abiraterone acetate, (3β)-17-(3-pyridinyl)androsta-5,16-dien-3-yl acetate, is a selective inhibitor of enzymes C17,20-lyase and 17α-hydroxylase on cytochrome P450 (CYP) 17. Abiraterone acetate is a prodrug. The compound is deacylated *in vivo* to Abiraterone which is absorbed in the GI tract after oral administration.

Abiraterone acetate has been approved with prednisone or prednisolone for the treatment of newly diagnosed high risk metastatic hormone sensitive prostate cancer (mHSPC) in adult men in combination with androgen deprivation therapy (ADT), the treatment of metastatic castration resistant prostate cancer (mCRPC) in adult men who are asymptomatic or mildly symptomatic after failure of androgen deprivation therapy in whom chemotherapy is not yet clinically indicated, and the treatment of mCRPCin adult men whose disease has progressed on or after a docetaxel-based chemotherapy regimen (Zytiga®: Summary of Product Characteristics, European Medicines Agency).

Abiraterone acetate as a pharmaceutically active compound has been disclosed in an International Patent Application WO 93/20097 A1 and is represented by a following structure:

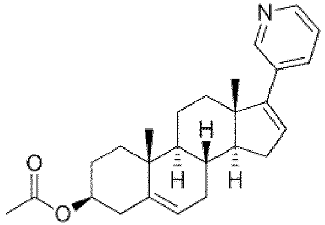

Abiraterone acetate is available in the form of tablets under the trade name Zytiga®, in two different doses (250 mg and 500 mg). A total of 1,000 mg is taken once a day.

Abiraterone acetate is a class IV compound according to Biopharmaceutical Classification System (BCS). Compounds in this category are characterized by low water solubility and low intestinal permeability. Absolute bioavailability of the active ingredient is unknown, but it is suggested that in the commercial form of the Zytiga® tablets in the fasted state it is unlikely for it to be higher than 10%.

Different strategies can be applied to increase bioavailability of the drug substance. One can mention approaches such as careful selection of excipients and incorporation of solubilizes into the formulation, reduction of active substance particle size to a micro- or even sub-micro range. Since commercially available Abiraterone acetate is usually provided in bulk in a micronized form with d(0,5) below 10 µm, only specific types of particle size reduction strategies could be applied. Incorporation of excipients that will act as solubilizers is much more simple solution.

Use of liquid self-microemulsifyng drug delivery systems (SEDDS) to increase bioavailability of lipophilic compounds is a known method adapted in several marketed products, e.g., Cyclosporine Neoral® soft gel capsules or Roaccutane® soft capsules with Isotretinoin.

SEDDS are isotropic mixtures of oils, surfactants, solvents and cosolvents/surfactants. SEDDS can be orally administered in soft or hard gelatin capsules and form fine relatively stable oil-in-water (o/w) emulsions upon aqueous dilution owing to the gentle agitation of the gastrointestinal fluids. The efficiency of oral absorption of the drug compound from the SEDDS depends on many formulation-related parameters, such as surfactant concentration, oil/surfactant ratio, polarity of the emulsion, droplet size and charge, all of which in essence determine the self-emulsification ability. Thus, only very specific pharmaceutical excipient combinations will lead to efficient self-emulsifying systems. (Gursoy RN, Benita S, Self-emulsifying drug delivery systems (SEDDS) for improved oral delivery of lipophilic drugs, Biomed Pharmacother. 2004 Apr; 58(3):173-82)*.*

Liquid or semisolid character of most SEDDS however causes problems with manufacturing of solid oral dosage forms that would promote the patient's compliance.

One approach taken to tackle this problem, described in WO 2016/162229, involves an additional process step to obtain a solid oral dosage form, a most commonly used step in which the liquid system is filled into capsules.

This process has many drawbacks including multiple stability risks and development limitations. Not all excipients used for development of SEDDS are compatible with market available soft or hard capsule shells. Only careful excipient selection can prevent stability issues like, e.g., significant changes in capsule appearance or physical migration of components between capsule filling and its shell/exterior or crosslinking of gelatin which is a main capsule shell material.

Other important challenge is active ingredient's crystallization during storage. Liquid SEDDS must be designed in a way to ensure that no active substance crystallization during product's shelf life will occur. International Patent Application WO 2016/162229 teaches obvious technical solution to this problem. SEDDS containing dissolved Abiraterone acetate is designed in a way that it is an unsaturated solution at room temperature, hence it keeps in a liquid form during storage. One needs to underline that this limits achievable formulation active substance load and excipient's choice hence decreases the number of possible ways to enhance active's substance bioavailability.

The problem of manufacturing a solid oral dosage form is approached in the same way in an International Patent Application WO 2014/009434. No proper technical solutions were presented for manufacturing solid dosage forms other than filling SEDDS in an unsaturated liquid form into capsules.

Solidification of self-emulsifying systems suitable for abiraterone acetate other than capsules filling is presented in International Patent Applications WO 2013/164473 and WO 2015/193380. Both teach to use a carrier as a way of manufacturing solid oral dosage form. However, processes applied therein are complex and multistep procedures.

WO 2013/164473 discloses using of solutions or dispersions of Abiraterone acetate in excipients that can form SEDDS systems after ingestion. These systems may be used in a liquid or semisolid form as such or filled into capsules in a standard way. Additionally, processes like spray drying or lyophilization of obtained compositions are shown as means to obtain powders. Application of this manufacturing technique for fully or partially dissolved Abiraterone acetate (e.g. nanosuspension) will lead to active substance crystallization, hence abilities to reconstitute system into emulsion will be limited by crystalline form's solubility and particle size that can change during the process. After this solidification procedure to tailor final product's properties, powders may be sprayed on or granulated with additional excipients to obtain solid mixtures that can be tableted.

An International Patent Application WO 2015/193380 discloses specific SEEDS systems for lipophilic compounds with log P at least 5 that can be administered as an encapsulated liquid or solidified by absorption on a solid carrier. Bulk of the described solid carrier consists of the absorbent excipient silicon dioxide. Additionally binder(s) and/or disintegrant(s) or even active substance itself might be added. Solid carrier is in form of granulate or tablets made of them. Thus, the main absorption step must be preceded by at least one additional manufacturing step of granulation and drying. Complexity of the manufacturing process is a disadvantage in terms of process control and costliness.

A technical solution described above allows for the active ingredient to be solubilized in the vehicle, act as a part of the solid carrier or be partly in the carrier and partly solubilized in the vehicle. This approach does not solve fully the problem of the poorly soluble crystalline active substance presence in the formulation. Solubilizing drug substance in the vehicle in the form of unsaturated solution is only a partial solution. Its limitations are the same as the ones described for the WO 2016/162229. Use of the lipophilic drug substance in its crystalline form to create SEDDS drastically reduces potential of the spontaneous emulsion formation and chances for it to dissolve thus limits possible increase in the bioavailability.

Absorption of the vehicle is achieved either by immersing the granulate into a vessel containing surplus of liquid vehicle or pouring a calculated amount of oil onto a bed of tablets rotating in some form of drum. Both described methods of carrying out the absorption process are obviously hard to control and poorly reproducible - endpoint of both processes will be always designated experimentally, and they will involve excess of the vehicle which will be lost during their course.

In this complex multistep process, no strategy preventing potential API recrystallization on the solid carrier's surface was presented. Additionally, granulation of the solid carrier containing porous silica and optional tableting thereof reduces available surface area of the solid carrier from which the vehicle might be absorbed. This feature of the proposed carrier prolongs the absorption time and makes the process susceptible to changes in the granulate particle size distribution.

Thus, there is still a need for development of drug forms comprising Abiraterone acetate characterized by high bioavailability as well as good chemical and physical stability.

### Disclosure of the invention

In the first aspect, provided is a pharmaceutical formulation comprising a solidified self-microemulsifyng drug delivery system (SEDDS) of Abiraterone acetate, wherein the system is completely absorbed into a mesoporous material that acts as a functional carrier for the system creating a stable solid oral formulation comprising Abiraterone acetate in a non-crystalline form.

In one preferable embodiment, the pharmaceutical formulation is obtainable by complete absorption of Abiraterone acetate liquid SEDDS system into a mesoporous material.

In one preferable embodiment, wherein the liquid SEDDS system is obtained by dissolving Abiraterone acetate in a mixture of solubilizer, water soluble surfactant, water insoluble surfactant and co-solvent/solubilizer.

In one preferable embodiment, the liquid SEDDS system is supersaturated.

In one more preferable embodiment, the liquid SEDDS system is supersaturated at temperatures at least up to 50°C.

In one more preferable embodiment, said solubilizer is a mixture of medium chain fatty acids mono- and diglycerides or medium chain fatty acids monoglycerides.

In one more preferable embodiment, wherein said water soluble surfactant is castor oil derivative or a mixture thereof.

In one more preferable embodiment, said water insoluble surfactant is propylene glycol fatty acid ester or a mixture thereof.

In one more preferable embodiment said co-solvent/solubilizer is glycol ether.

In one more preferable embodiment, a mixture of solubilizer, water soluble surfactant, water insoluble surfactant and co-solvent/ solubilizer comprises said components in the proportion, in weight percent, 5-15 : 5-30 : 5-15 : 5-15.

In one more preferable embodiment, wherein a mixture of solubilizer, water soluble surfactant, water insoluble surfactant and co-solvent/solubilizer comprises said components in the proportion, in weight percent, about 10 : 20 : 10 : 10.

In one preferable embodiment a mesoporous material is selected from the group comprising mesoporous magnesium aluminum silicate, mesoporous calcium silicate and mesoporous silica.

In one preferable embodiment, a mesoporous material is mesoporous silica.

In one more preferable embodiment, a mesoporous material constitutes, in weight percent, not more than 40% of the SEDDS formulation.

In one more preferable embodiment, a mesoporous carrier constitutes, in weight percent, 30-40% of the SEDDS formulation.

In one more preferable embodiment, the active substance load on a mesoporous material is about 10 wt.-%.

In the second aspect, provided is a process of preparing the pharmaceutical formulation comprising solidified self-microemulsifyng drug delivery system (SEDDS) of Abiraterone acetate, comprising the steps of:
a) Preparation of surfactants and solubilizers mixture by mixing and heating its components at elevated temperature in the range 70-80°C,
b) Adding and dissolving active ingredient and optionally antioxidant and/or preservative while continuously stirring the mixture until the clear solution is obtained,
c) Adding mesoporous material to the mixture while continuously stirring and maintaining the temperature in the range 70-80°C until whole amount of SEDDS is completely absorbed.

In the third aspect, provided is a use of the pharmaceutical formulation according to Claim 1 for manufacturing solid oral dosage forms comprising Abiraterone acetate as an active ingredient in a non-crystalline form.

In the fourth aspect, provided is a solid oral dosage form comprising the pharmaceutical formulation comprising solidified self-microemulsifyng drug delivery system (SEDDS) of Abiraterone acetate as an active ingredient in a non-crystalline form absorbed into a mesoporous material and additional pharmaceutically acceptable excipients.

In one preferable embodiment, the solid oral dosage form is in the form of powder, granules, sachets, capsules or tablets.

In one more preferable embodiment, in the solid oral dosage form, the additional pharmaceutically acceptable excipients are selected from a group comprising antioxidants, preservatives, lubricants, glidants, sweeteners, or taste-masking agents.

### Detailed description of the invention

In the first aspect, as mentioned above, the present invention provides a pharmaceutical formulation comprising a solidified self-microemulsifyng drug delivery system (SEDDS) of Abiraterone acetate, wherein the system is completely absorbed into a mesoporous material that acts as a functional carrier for the system creating a stable solid oral formulation comprising Abiraterone acetate in a non-crystalline form.

The term "SEDDS" as used herein is intendent to mean a self-microemulsifyng drug delivery system comprising a mixture of solubilizers, surfactants and cosolvents, intended to dissolve or suspend an active ingredient, that creates a stable microemulsion upon contact with water medium, particularly with gastric fluids.

The term "SEDDS formulation" as used herein is intended to mean a pharmaceutical composition comprising the above described SEDDS system including an active ingredient absorbed into a carrier and optionally additional pharmaceutically acceptable agents that enhance the composition's stability, eg. antioxidants and/or preservatives.

Preferably, the pharmaceutical formulation of the invention is obtainable by complete absorption of Abiraterone acetate liquid SEDDS system into a mesoporous material.

Further preferably, the liquid SEDDS system is obtained by dissolving Abiraterone acetate in a mixture of solubilizer, water soluble surfactant, water insoluble surfactant and co-solvent/solubilizer.

The liquid SEDDS system used for preparing the SEDDS formulation is preferably supersaturated. More preferably, the formulation is supersaturated at temperatures at least up to 50°C, preferably 70°C, more preferably 75-80°C, but in one embodiment, it can be saturated up to 45°C.

Solubilizer is preferably a mixture of medium chain fatty acids mono- and diglycerides or medium chain fatty acids monoglycerides, so it is preferable that the solubilizer has a typical lipid character.

Water soluble surfactant is preferably castor oil derivative or a mixture thereof.

Water insoluble surfactant is preferably propylene glycol fatty acid ester or a mixture thereof.

Co-solvent/solubilizer is preferably glycol ether.

In a preferred embodiment of the invention, Abiraterone acetate is dissolved in a mixture comprising solubilizer, water soluble surfactant, water insoluble surfactant and co-solvent/solubilizer in the proportion, in weight percent, 5-15 : 5-30 : 5-15 : 5-15.

In more preferred embodiment of the invention, a mixture comprises solubilizer, water soluble surfactant, water insoluble surfactant and co-solvent/solubilizer in the proportion, in weight percent, about 10 : 20 : 10 : 10.

Mesoporous carrier is selected from the group comprising mesoporous magnesium aluminum silicate (e.g. Neusiline US2), mesoporous calcium silicate (e.g. Florite PS200) and mesoporous silica (e.g. Syloid XDP 3150).

In the preferred embodiment of the invention, mesoporous carrier is mesoporous silica.

In the preferred embodiment of the invention, mesoporous carrier constitutes, in weight percent, not more than 40% of the SEDDS formulation.

Preferably, the mesoporous carrier constitutes, in weight percent, about 30-40% of the SEDDS formulation.

In one embodiment, active substance load into a mesoporous carrier is about 10 wt.-%.

The solidified self-microemulsifyng drug delivery system (SEDDS) formulation comprising Abiraterone acetate in a non-crystalline form can be prepared in the process comprising the steps of, forming the second aspect of the present invention:
a) Preparation of surfactants and solubilizers mixture by mixing and heating its components at elevated temperature in the range 70-80°C,
b) Adding and dissolving an active ingredient and optionally antioxidant/preservative while continuously stirring the mixture until the clear solution is obtained,
c) Adding a mesoporous carrier to the mixture while continuously stirring and maintaining the temperature in the range 70-80°C until a whole amount of SEDDS is completely absorbed.

One can distinguish two crucial stages of the solidified SEDDS formulation manufacturing process.

First, preparing liquid solution of Abiraterone acetate in the mixture of SEDDS' components. This step requires controlled heating of the mixture up to 70-80°C and keeping components in the proper weight ratios to guarantee process and product reproducibility.

Second crucial stage is complete absorption of the liquid SEDDS into the mesoporous carrier. Incomplete absorption might result in the Abiraterone acetate crystallization on the carrier surface. Therefore process must be continued for some time (up to 1.5 h depending on the excipients used) after liquid phase disappearance at temperature at which the active substance can be completely dissolved in the solution. This step of additional conditioning of the product ensures absorption and physically stable non-crystalline form of Abiraterone acetate in the formulation.

Simple strategy comprising mixing the SEDDS at the temperature specific for the given liquid system and subsequent conditioning of the obtained solid powder ensures complete absorption and prevents Abiraterone acetate's crystallization. Even active substance supersaturated solution is preserved in the carriers' pores in the non-crystalline form after reconstitution of the SEDDS system and is not limited by Abiraterone acetate crystalline form's solubility.

Preparation process according to the invention is a "one-pot" operation that shows an advantage over the prior art solutions in its actual simplicity. Limited number of technologically simple operations guaranties manufacturing process reproducibility and final product's consistent high quality.

Mesoporous carrier used in the present invention plays a role of not only a solidifying agent but also prevents possible crystallization of Abiraterone acetate from the liquid SEDDS (even in the supersaturated state) during long-term storage of the final product. This feature enables stable over time and reproducible SEDDS reconstitution in the GI tract upon contact with water medium after administration of a final dosage form to a patient. The above described functionality is not a result of just a single carrier feature but is a combination of multiple elements such as carrier's porous structure, effect of simple but efficient manufacturing process ensuring a non-crystalline form of Abiraterone acetate in the product as well as liquid formulation composition. The above mentioned features enable usage and the properties' preservation of the supersaturated liquid SEDDS according to the invention.

The supersaturated state of solidified SEDDS likely plays also a role in the observed bioavailability increase relative to Zytiga® 250 mg tablets *(*Eur J Pharm Biopharm. 2015 Feb; 90:1-7*).*

As mentioned above, one of the key features of the invention is the capability of preserving a supersaturated state of SEDDS wherein Abiraterone acetate is in a non-crystalline form by incorporating the system into the mesoporous structure of a carrier.

Self-emulsifying system containing Abiraterone acetate, depending on its concentration, is supersaturated at temperatures at least up to 50°C, preferably 70°C, more preferably 75-80°C. It means that below the specific saturation temperature system is a suspension of crystalline active substance in excipients mixture.

Concentration dependent saturation temperature for the specific SEDDS can be established visually by observing a system while heating it. Above the saturation temperature suspension becomes a clear solution.

SEDDS containing Abiraterone acetate can be fully absorbed into the mesoporous carrier only as a clear solution (in a non-saturated state) at temperatures exceeding saturation point above which an active substance is completely dissolved in the system. Only in this particular case supersaturation can be preserved in the final product and enhance a suitable Abiraterone acetate load in the formulation. In contrast to that, any attempts of incorporating SEDDS system in the form of suspension lead to a partial active substance absorption in the porous structure and therefore will result in a presence of crystalline Abiraterone acetate in the final formulation.

SEDDS formulation absorbed into a mesoporous carrier according to invention can be used for manufacturing stable solid oral formulations comprising Abiraterone acetate in a non-crystalline form.

Solid oral dosage forms according to invention comprise SEDDS formulation comprising Abiraterone acetate as an active ingredient in a non-crystalline form absorbed into a mesoporous carrier and additional pharmaceutically acceptable excipients.

Said solid oral dosage form can be in the form of powder, granules, capsules, sachets or tablets.

In one embodiment of the invention, the SEDDS formulation absorbed into a mesoporous carrier can be used as such for filling capsules or sachets.

In the alternative embodiment of the invention, the SEDDS formulation absorbed into a mesoporous carrier can be admixed with additional pharmaceutically acceptable excipients and further processed into granules or compressed into tablets.

Solid oral dosage form can further comprise, besides SEDDS formulation according to invention, some additional pharmaceutically acceptable excipients, eg. antioxidants, preservatives, lubricants, glidants, sweeteners, taste-masking agents, etc. They can be added to SEDDS formulation prior to its absorption on a mesoporous carrier, like antioxidants and preservatives, or to a formulation after SEDDS's absorption into a mesoporous carrier, like lubricants, glidants, sweeteners, or taste-masking agents.

Finally, a solid dosage form is prepared by distribution of the solidified SEDDS formulation into sachets or compression into tablets or filling the capsules with it.

That final step may be preceded by addition of further optional pharmaceutically acceptable excipients to give appropriate properties to the final dosage form, improving powder specific physical features, e.g. granules flowability, tablet hardness, taste, appearance, etc., after absorption process is completed.

All mentioned processes can be done using customary procedures known in the art of pharmaceutical formulation.

SEDDS formulation according to the invention is characterized by increased bioavailability in comparison to Zytiga® 250 mg tablets and a good chemical and physical stability.

The invention is illustrated by the following examples.

### Examples

### Example 1

200 g batch formulation of Abiraterone acetate and SEDDS system components in amounts listed in the Table below was prepared in accordance to a following manufacturing process.

| No. | Substance | Amount | |
|---|---|---|---|
| | | [mg/50 mg dose] | [%] |
| 1 | Abiraterone acetate (active ingredient) | 50.02 | 10.25 |
| 2 | Capryol 90 (surfactant) | 50.07 | 10.26 |
| 3 | Cremophor EL (surfactant) | 99.75 | 20.44 |
| 4 | Transcutol HP (co-solvent/solubilizer) | 50.07 | 10.26 |
| 5 | Capmul MCM (solubilizer) | 50.02 | 10.25 |
| 6 | Syloid XDP 3150 (carrier) | 187.68 | 38.46 |
| 7 | BHT (antioxidant) | 0.39 | 0.08 |
| **Total** | | 488 | 100 |

Capmul MCM (20.50 g), Capryol 90 (20.50 g), Cremophor EL (40.88 g) and Transcutol HP (20.52 g) were weighed into a 1000 ml beaker and placed on a heating plate. The mixture was heated up to 70-80°C while stirring (overhead mechanical stirrer equipped with a propeller, 4-bladed impeller). After a clear solution was obtained, Abiraterone acetate (20.50 g) and BHT (0.16 g) were added and the resulting mixture was continuously stirred until the clear, yellowish solution was obtained. Syloid XDP 3150 (76.92 g) was added while continuously stirring obtained solution and maintaining the temperature in a range 70-80°C until whole amount of SEDDS was completely absorbed (up to 1.5 h).

Obtained powder was analyzed for the active ingredient's impurity content (HPLC) and crystallinity (XRPD).

Process resulted in about 200 g of free flowing powder of SEDDS formulation. A final dosage form was prepared by filling capsules bodies No. 00 with weighted portions of the powder comprising 50 mg dose of Abiraterone acetate. Capsules were packed in Alu-Alu blisters.

### Example 2

Example 2 was prepared in the same manner as Example 1 using the ingredients and amounts listed in the table below.

| No. | Substance | Amount | |
|---|---|---|---|
| | | [mg/50 mg dose] | [%] |
| 1 | Abiraterone acetate (active ingredient) | 50.02 | 10.25 |
| 2 | Capryol 90 (surfactant) | 62.61 | 12.83 |
| 3 | Cremophor RH40 (surfactant) | 74.66 | 15.30 |
| 4 | Transcutol HP (co-solvent/solubilizer) | 62.61 | 12.83 |
| 5 | Capmul MCM (solubilizer) | 50.02 | 10.25 |
| 6 | Syloid XDP 3150 (carrier) | 187.68 | 38.46 |
| 7 | BHT | 0.39 | 0.08 |
| **Total** | | 488 | 100 |

### Example 3

Example 3 was prepared in the same manner as Example 1 using the ingredients and amounts listed in the table below.

| No. | Substance | Amount | |
|---|---|---|---|
| | | [mg/50 mg dose] | [%] |
| 1 | Abiraterone acetate (active ingredient) | 50.02 | 10.25 |
| 2 | Capryol 90 (surfactant) | 50.07 | 10.26 |
| 3 | Cremophor EL (surfactant) | 99.75 | 20.44 |
| 4 | Transcutol HP (co-solvent/solubilizer) | 50.07 | 10.26 |
| 5 | Capmul MCM (solubilizer) | 50.02 | 10.25 |
| 6 | Florite PS 200 (carrier) | 187.68 | 38.46 |
| 7 | BHT (antioxidant) | 0.39 | 0.08 |
| **Total** | | 488 | 100 |

### Example 4

Example 4 was prepared in the same manner as Example 1 using the ingredients and amounts listed in the table below.

| No. | Substance | Amount | |
|---|---|---|---|
| | | [mg/50 mg dose] | [%] |
| 1 | Abiraterone acetate (active ingredient) | 50.02 | 10.25 |
| 2 | Capryol 90 (surfactant) | 62.61 | 12.83 |
| | Cremophor EL (surfactant) | 37.33 | 7.65 |
| 3 | Cremophor RH40 (surfactant) | 37.33 | 7.65 |
| 4 | Transcutol HP (co-solvent/solubilizer) | 62.61 | 12.83 |
| 5 | Capmul MCM (solubilizer) | 50.02 | 10.25 |
| 6 | Florite PS 200 (carrier) | 187.68 | 38.46 |
| 7 | BHT | 0.39 | 0.08 |
| **Total** | | 488 | 100 |

### Example 5

Example 5 was prepared in the same manner as Example 1 using the ingredients and amounts listed in the table below.

| No. | Substance | Amount | |
|---|---|---|---|
| | | [mg/50 mg dose] | [%] |
| 1 | Abiraterone acetate (active ingredient) | 50.02 | 10.25 |
| 2 | Capryol 90 (surfactant) | 50.07 | 10.26 |
| 3 | Cremophor EL (surfactant) | 99.75 | 20.44 |
| 4 | Transcutol HP (co-solvent/solubilizer) | 50.07 | 10.26 |
| 5 | Capmul C8 (solubilizer) | 50.02 | 10.25 |
| 6 | Neusiline US2 (carrier) | 187.68 | 38.46 |
| 7 | BHT (antioxidant) | 0.39 | 0.08 |
| **Total** | | 488 | 100 |

### Example 6

Example 6 was prepared in the same manner as Example 1 using the ingredients and amounts listed in the table below.

| No. | Substance | Amount | |
|---|---|---|---|
| | | [mg/50 mg dose] | [%] |
| 1 | Abiraterone acetate (active ingredient) | 50.02 | 10.25 |
| 2 | Lauroglycol 90 (surfactant) | 62.61 | 12.83 |
| 3 | Cremophor RH40 (surfactant) | 74.66 | 15.30 |
| 4 | Transcutol HP (co-solvent/solubilizer) | 62.61 | 12.83 |
| 5 | Capmul MCM C10 (solubilizer) | 50.02 | 10.25 |
| 6 | Neusiline US2 (carrier) | 187.68 | 38.46 |
| 7 | BHT | 0.39 | 0.08 |
| **Total** | | 488 | 100 |

### Example 7

Example 7 was prepared in the same manner as Example 1 using the ingredients and amounts listed in the table below.

| No. | Substance | Amount | |
|---|---|---|---|
| | | [mg/50 mg dose] | [%] |
| 1 | Abiraterone acetate (active ingredient) | 50.02 | 10.25 |
| 2 | Capryol 90 (surfactant) | 50.07 | 10.26 |
| 3 | Cremophor ELP (surfactant) | 99.75 | 20.44 |
| 4 | Transcutol HP (co-solvent/solubilizer) | 50.07 | 10.26 |
| 5 | Capmul 471 (solubilizer) | 50.02 | 10.25 |
| 6 | Florite PS 200 (carrier) | 187.68 | 38.46 |
| 7 | BHT (antioxidant) | 0.39 | 0.08 |
| **Total** | | 488 | 100 |

### Example 8

Example 8 was prepared in the same manner as Example 1 using the ingredients and amounts listed in the table below.

| No. | Substance | Amount | |
|---|---|---|---|
| | | [mg/50 mg dose] | [%] |
| 1 | Abiraterone acetate (active ingredient) | 50.02 | 10.25 |
| 2 | Labrafac PG (surfactant) | 62.61 | 12.83 |
| | Cremophor EL (surfactant) | 37.33 | 7.65 |
| 3 | Cremophor RH40 (surfactant) | 37.33 | 7.65 |
| 4 | Transcutol HP (co-solvent/solubilizer) | 62.61 | 12.83 |
| | Capmul MCM C10 (solubilizer) | 37.54 | 7.70 |
| 5 | Capmul MCM (solubilizer) | 12.48 | 2.56 |
| 6 | Syloid XDP 3150 (carrier) | 187.68 | 38.46 |
| 7 | BHT | 0.39 | 0.08 |
| **Total** | | 488 | 100 |

### Example 9

Example 9 was prepared in the same manner as Example 1 using the ingredients and amounts listed in the table below.

| No. | Substance | Amount | |
|---|---|---|---|
| | | [mg/50 mg dose] | [%] |
| 1 | Abiraterone acetate (active ingredient) | 50.02 | 10.25 |
| 2 | Capryol 90 (surfactant) | 62.61 | 12.83 |
| | Cremophor EL (surfactant) | 37.33 | 7.65 |
| 3 | Cremophor RH40 (surfactant) | 37.33 | 7.65 |
| 4 | Transcutol HP (co-solvent/solubilizer) | 62.61 | 12.83 |
| | Capmul MCM 471 (solubilizer) | 25.01 | 5.13 |
| 5 | Capmul MCM (solubilizer) | 25.01 | 5.13 |
| 6 | Syloid XDP 3150 (carrier) | 187.68 | 38.46 |
| 7 | BHT | 0.39 | 0.08 |
| **Total** | | 488 | 100 |

### Results

### A. Bioavailability

Formulation of Example 1 has been found to show increased relative bioavailability in comparison to Zytiga® 250 mg tablets. Comparison was conducted in a PK study on Sprague-Dawley rats chosen as an animal model. Given doses were calculated in respect to abiraterone acetate and administered *p*.*o*. each to a group of 5 animals. Blood samples were taken predose and 0.5, 1, 1.5, 2, 3, 4 and 6 h after administration and placed in tubes containing anticoagulant K₂EDTA. Blood plasma was isolated by centrifugation and samples were stored at -80°C. Plasma samples were analyzed by UHPLC-MS/MS method to access Abiraterone concentration.

| Formulation | Administered dose of Abiraterone acetate [mg/kg] | Average AUC₍₀₋₆ₕ₎ [h•ng/ml] | Average AUC₍₀₋₆ₕ₎ per administered dose [(h•ng/ml)/(mg/kg)] | AUC Example 1 vs. Zytiga® 250 mg |
|---|---|---|---|---|
| Example 1 | 22 | 203.0 | 9.2 | 3.3 |
| Zytiga® 250 mg | 79 | 222.4 | 2.2 | |

### B. Stability study

Stability study has been performed for Example 1 capsules packed in Alu-Alu blisters. Blisters were placed in stability chambers under normal conditions (25°C, 75%RH). Capsules were analyzed by HPLC method after 1, 3 and 6 month and compared to the starting point (directly after manufacturing).

| Conditions | Example 1 | | | |
|---|---|---|---|---|
| | start | 1 month 25°C 75% RH | 3 months 25°C 75% RH | 6 months 25°C 75% RH |
| Sum of impurities Limit < 2.0% | 0.06% | 0.13% | 0.17% | 0.35% |

Potential recrystallisation of Abiraterone acetate has been studied by X-Ray Powder Diffraction method (XRPD). Diffractograms were recorded using Mini Flex powder diffractometer (Rigaku) with CuKα radiation (λ=1.54060 Ǻ), with a sampling width Δ2θ = 0.02° and scanning rate 1.00°/min.

XRPD diffractograms were recorded and analyzed within a range 2-40° 2θ.

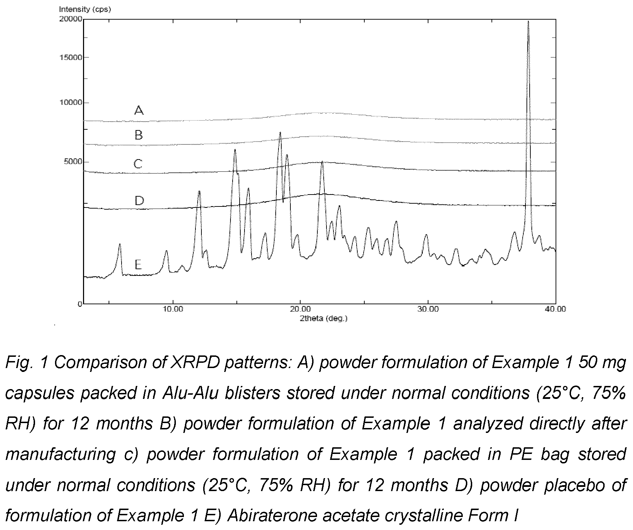

Fig. 1 shows XRPD patterns of powder formulation of Example 1 50 mg capsules packed in Alu-Alu blisters stored under normal conditions (25°C 75% RH) for 12 months, powder formulation of Example 1 analyzed directly after manufacturing, powder formulation of Example 1 packed in a PE bag stored under normal conditions (25°C, 75% RH) for 12 months, powder placebo of formulation of Example 1 and Abiraterone acetate crystalline Form I. No crystalline Abiraterone acetate was detected in the formulation stored for 12 months.

Obtained results confirm carrier's functional role in preserving an active ingredient in a non-crystalline form inside its mesoporous structure.

## Claims

1. A pharmaceutical formulation comprising a solidified self-microemulsifyng drug delivery system (SEDDS) of Abiraterone acetate, wherein the system is completely absorbed into a mesoporous material that acts as a functional carrier for the system creating a stable solid oral formulation comprising Abiraterone acetate in a non-crystalline form.

2. The pharmaceutical formulation according to Claim 1, obtainable by complete absorption of Abiraterone acetate liquid SEDDS system into a mesoporous material.

3. The pharmaceutical formulation according to Claim 1 or 2, wherein the liquid SEDDS system is obtained by dissolving Abiraterone acetate in a mixture of solubilizer, water soluble surfactant, water insoluble surfactant and co-solvent/solubilizer.

4. The pharmaceutical formulation according to any of claims 1-3, wherein the liquid SEDDS system is supersaturated, preferably it is saturated at temperatures at least up to 50°C.

5. The pharmaceutical formulation according to any of claims 1-4, wherein said solubilizer is a mixture of medium chain fatty acids mono- and diglycerides or medium chain fatty acids monoglycerides.

6. The pharmaceutical formulation according to any of claims 1-5, wherein said water soluble surfactant is castor oil derivative or a mixture thereof.

7. The pharmaceutical formulation according to any of claims 1-6, wherein said water insoluble surfactant is propylene glycol fatty acid ester or a mixture thereof.

8. The pharmaceutical formulation according to any of claims 1-7, wherein said co-solvent/solubilizer is glycol ether.

9. The pharmaceutical formulation according to any of claims 1-8, wherein a mixture of solubilizer, water soluble surfactant, water insoluble surfactant and co-solvent/ solubilizer comprises said components in the proportion, in weight percent, 5-15 : 5-30 : 5-15 : 5-15.

10. The pharmaceutical formulation according to Claim 9, wherein a mixture of solubilizer, water soluble surfactant, water insoluble surfactant and cosolvent/solubilizer comprises said components in the proportion, in weight percent, about 10 : 20 : 10 : 10.

11. The pharmaceutical formulation according to any of claims 1-10, wherein a mesoporous material is selected from the group comprising mesoporous magnesium aluminum silicate, mesoporous calcium silicate and mesoporous silica.

12. The pharmaceutical formulation according to any of claims 1-11, wherein a mesoporous material is mesoporous silica.

13. The pharmaceutical formulation according to any of claims 1-12, wherein a mesoporous material constitutes, in weight percent, not more than 40% of the SEDDS formulation.

14. The pharmaceutical formulation according to any of claims 1-13, wherein a mesoporous constitutes, in weight percent, 30-40% of the SEDDS formulation.

15. The pharmaceutical formulation according to any of claims 1-14, wherein the active substance load on a mesoporous material is about 10 wt.-%.

16. A process of preparing the pharmaceutical formulation comprising solidified self-microemulsifyng drug delivery system (SEDDS) of Abiraterone acetate, comprising the steps of:
a) Preparation of surfactants and solubilizers mixture by mixing and heating its components at elevated temperature in the range 70-80°C,
b) Adding and dissolving active ingredient and optionally antioxidant and/or preservative while continuously stirring the mixture until the clear solution is obtained,
c) Adding mesoporous material to the mixture while continuously stirring and maintaining the temperature in the range 70-80°C until whole amount of SEDDS is completely absorbed.

17. An use of the pharmaceutical formulation according to Claim 1 for manufacturing solid oral dosage forms comprising Abiraterone acetate as an active ingredient in a non-crystalline form.

18. A solid oral dosage form comprising the pharmaceutical formulation comprising solidified self-microemulsifyng drug delivery system (SEDDS) of Abiraterone acetate as an active ingredient in a non-crystalline form absorbed into a mesoporous material and additional pharmaceutically acceptable excipients.

19. The solid oral dosage form according to Claim 18 in the form of powder, granules, sachets, capsules or tablets.

20. The solid oral dosage form according to Claims 18 or 19, wherein additional pharmaceutically acceptable excipients are selected from a group comprising antioxidants, preservatives, lubricants, glidants, sweeteners, or taste-masking agents.
